# EUROPEAN PATENT APPLICATION

(11) **EP 3 409 224 A1**
(43) Date of publication of application: **05.12.2018**
(21) Application number: 18184608.0
(22) Date of filing: 03.04.2015
(51) Int. Cl.: A61B 17/62, A61B 17/66

(54) **F.A. MATSUKATOV COMPRESSION-DISTRACTION APPARATUS AND ASSEMBLY FOR REPOSITIONING SAME**

(30) Priority: 04.04.2014 KZ 20140443
(62) Divisional of application: 15773347.8
(71) Applicant: Tovarishchestvo S Ogranichennoy Otvetstvennostiyu, Kostanay 110000 (KZ)
(72) Inventor: MATSUKATOV, Feodor Alekseevich, 640014 Kurgan (RU)
(74) Representative: Isarpatent

(57) **Abstract**

This group of inventions relates to the field of medicine, and more particularly to traumatology and orthopaedics, and can be used for treating patients with damaged or diseased limb bones. The present compression-distraction apparatus comprises: supports in the form of rings provided with apertures and interconnected by threaded rods; bone anchors; and also fastening members, wherein at least one of the supports is comprised of coaxial inner and outer parts mounted so as to be capable of rotating relative to each other, the coaxial outer part of the support in question having, on its outer face, lugs with openings and being formed from rigidly interconnected curved segments. The curved segments of the coaxial outer part of the support have toothed rings on the inner face, and the coaxial inner part of the support has a groove on its outer face, in which the toothed rings of the curved segments of the coaxial outer part of the support are mounted; the coaxial inner part of the support is provided with apertures, at least one of which communicates with the aforementioned groove of the coaxial inner part of the support and in which a key with a toothed ring is mounted so as to be capable of interacting with the toothed ring of a curved segment of the coaxial outer part of the support. Also envisaged are novel embodiments of repositioning assemblies. The inventions provide a simplified design for a compression-distraction apparatus and repositioning assemblies, greater ease of installation and increased repositioning efficiency and fixation stability.

## Description

These inventions relate to the field of medicine and, in particular, to traumatology and orthopedic surgery, and can be used for treatment of patients with damaged or diseased limb bones.

The G.A. Ilizarov apparatus for transosseous osteosynthesis is known in the art (USSR Inventor's Certificate No. 1055499, IPC A61B17/18, publ. 23.11.1983). It comprises supports in the form of closed and/or open rings having apertures and connected via threaded rods, bone fixators, and fastening members. The bone fixators are made in the form of pins and are fixed on the supports by pin holders. The supports form units for fixation of a proximal bone fragment and a distal bone fragment. The supports are made of two interconnected arcuate segments. The apparatus is provided with an additional support in the form of a ring connected to the units for fixation of a proximal fragment and a distal fragment via threaded rods and repositioning assemblies. The repositioning assemblies are arranged on the additional support performing the function of rotatable guide and consist of bars, each provided on its one end with a flange having a threaded hole which axis coincides with the bar surface plane, braces with threaded ends, and washers having a prismatic slot tangential to the hole. Several designs of the repositioning assembly composed of the above parts are provided for. One repositioning assembly comprises a threaded strut fixed in the holes of two bars secured in the threaded holes of the other bars rigidly connected to the additional support via fastening members. A threaded strut is installed into the threaded hole of another bar connected to the support via a threaded rod. Another repositioning assembly, which is used for elimination of angular shift, comprises bars hingedly interconnected and connected to the threaded rods of the supports.

When using this apparatus for treating multi-component deformations of limb segments, including rotary deformities, it is necessary to install multi-part functional units, which hampers osteosynthesis, and in a number of case repeated remounting of the apparatus is required.

The compression-distraction apparatus (Patent RU 2357699, IPC A61B17/66, publ. 10.06.2009) has been taken as the closest analogous solution. It comprises supports in the form of closed and/or open rings having holes and connected via threaded rods, bone fixators as well as fastening members in the forms of washers, nuts and fastening bolts, at least one of the supports being composed of a coaxial inner part and a coaxial outer part that are arranged with the possibility of being rotated by a wrench with a toothed ring. The external end of the support coaxial outer part is provided with lugs having holes, and the part itself is formed of arcuate segments rigidly interconnected. The arcuate segments of the coaxial outer part of the support are provided with slots on their internal end faces. The external end of the support coaxial inner part is provided with toothed ring arranged in the slots of the arcuate segments of the support coaxial outer part. The hole of at least one of the lugs is in communication with the slot of the support coaxial outer part and is provided with a wrench with a toothed ring capable of cooperating with the toothed ring of the support coaxial inner part. The arcuate segments of the support coaxial outer parts are provided with L-curved ends and with a matching rectangular slot on the outer surface, a matching recess for a threaded bush on the inner surface, and a matching slot coaxial with the bush opening at the end. The ends of every segment of the support coaxial outer part are connected with a fixing brace that is provided on its ends with protrusions corresponding to the slots on the outer surface of the L-curved ends of segments of the support coaxial outer parts and with a slot on the end inner surface for a drive screw having circular recess in the head basis.

The repositioning assemblies discussed in the specification to Patent RU 2357699 (IPC A61B 17/66, publ. 10.06.2009) have been selected as the closest analogous solution of the repositioning assemblies of the claimed compression-distraction apparatus. They are made in the form of sleeves that are hingedly connected via threaded shanks to fork-shaped brackets attached to the apparatus threaded rods by fastening members. These sleeves are movably and pivotally arranged on a threaded strut, and the threaded strut is installed into the hole of a slider having a T-sectioned longitudinal slot on the side opposite to the hole. The slider has protrusions for rectangular slots on the end outer surface of the arcuate segments of the support coaxial outer parts. It is arranged on the support in the rectangular slots on the end outer surface of the arcuate segments of the support coaxial outer parts. A drive screw is installed into the T-sectioned slot of the slider and fixed by a plug with a recess. The drive screw is also arranged in the coaxial slot on the end face of the L-shaped ends of the arcuate segments of the support coaxial outer part and the coaxial threaded bush.

This apparatus expands the functional capabilities while treating patients having short limb segments in combination with their multicomponent deformity. However, it is not sufficiently effective for repositioning of bone fragments due to that the presence of a toothed ring on the arcuate segments of the support coaxial inner parts hampers rotational movements, since the apparatus main parts, including bone fixators, are attached on the same segments. In addition, the complex configuration of the arcuate segment ends in the support coaxial outer part is irrational, which complicates both their manufacturing and the apparatus mounting. The repositioning assemblies in this construction are fixed in the connection points of the arcuate segments of the arcuate segments of the support coaxial outer part, which limits their utilization efficiency during repositioning of bone fragments. The known repositioning assemblies are difficult to mount and use during repositioning. The use of the fork-shaped brackets for connecting the threaded strut to the apparatus threaded rods cannot achieve sufficient fixation stability, since play is created. As one more disadvantage, limited observability of X-ray pictures due to bulkiness of the repositioning assembly sliders may be mentioned.

The present invention is based on the task of designing a compression-distraction apparatus and its repositioning assemblies having broad functional capabilities, but simpler in their construction and easier in mounting and use, stable during fixation and having higher utilization efficiency during repositioning bone fragments as well as enabling to select their most optimal arrangement.

The technical effect is simplification of the constructions of the compression-distraction apparatus itself and its repositioning assemblies, simplification of their mounting and higher repositioning efficiency and fixation stability.

In order to achieve the above technical effect in a compression-distraction apparatus comprising supports in the form of closed or open rings having holes and connected via threaded rods, bone fixators and fastening members, at least one of said supports being composed of coaxial inner and outer parts arranged with the possibility of being pivoted relative to each other by a wrench provided with a toothed ring, the coaxial outer part of the support being provided with lugs, in which holes are made, on its external end face and formed of arcuate segments rigidly interconnected, *according to this invention,* the arcuate segments of the support coaxial outer part are provided with toothed rings on its internal end, and the support coaxial inner part is provided with a slot on its external end face, wherein the toothed rings of the arcuate segments of the support coaxial outer part are arranged, the support coaxial inner part is provided with holes, at least one such hole being in communication with the slot made in the support coaxial inner part and receiving a wrench with a toothed ring capable of communicating with the toothed ring of an arcuate segment of the support coaxial outer part.

According to preferable embodiments of the compression-distraction apparatus, the supports are connected via threaded rods and repositioning assemblies.

Being mainly coaxial, the inner part of the support is composed of arcuate segments which ends are provided with stepped protrusions overlapping matched stepped protrusions with holes, which are made on the adjacent arcuate segment of the support coaxial inner part and rigidly fixed by fastening members.

According to another embodiment, the support coaxial inner part of the compression-distraction apparatus in the form of an open ring is made solid, and the slot on its external end face is made T-sectioned with protrusions facing each other, the arcuate segments of the support coaxial outer part are provided with slots in the toothed ring bases on both sides for forming T-shaped protrusions corresponding to the said slot made in the support coaxial inner part.

Preferably, the arcuate segments of the support coaxial outer part are provided on their ends with stepped protrusions with holes. which overlap the matched stepped protrusions with holes of the adjacent arcuate segment of the support coaxial outer part, thus forming lugs, and are rigidly fixed via fastening members.

Furthermore, the repositioning assemblies of the compression-distraction apparatus may be made in the form of sleeves which are hingedly connected with the use of their toothed shanks to L-shaped brackets fixed on the apparatus threaded rods via fastening members, said sleeves being arranged on a threaded strut with the possibility of moving linearly and pivoting, and the threaded strut is arranged with the possibility of moving linearly and pivoting in the slider hole and is fixed via fastening members, the slider is provided on the side opposite to the said hole with a T-sectioned longitudinal slot on which ridges a guide is arranged via longitudinal slots made on its lateral sides, the end of the T-sectioned slot of the slider is provided with a plug with a recess, and a drive screw is arranged in the T-sectioned slot of the slider and in the longitudinal threaded channel made in the guide, which drive screw is fixed via the plug with recess, and the slider is attached to the support via an external shank made on the guide and arranged in the support hole and via a fastening member.

According to preferred embodiments of the compression-distraction apparatus, each of the repositioning assemblies comprises: a threaded strut; a first and a second L-shaped brackets arranged on the threaded strut with the possibility of moving linearly and pivoting and fixed via fastening members, the first L-shaped bracket being provided with threaded holes in both flanges and connected to an apparatus threaded rod via a fastening member; a bar having two longitudinal protrusions on its opposite lateral sides, a lug on one its end, and a toothed cut piece on the other end; a fork provided with longitudinal recesses inside the end portions of both flanges and coaxial through holes made in both flanges for a wrench having a toothed ring, the bar being arranged inside the fork with the possibility of moving in such a way that its longitudinal protrusions cooperate with the recesses in the fork flanges, and the toothed cut piece cooperates with the toothed ring of the wrench arranged in the through holes of the fork flanges; the second L-shaped bracket being provided with a hole for the threaded strut on one flange and with an external threaded shank on the other flange, and the threaded shank of the second L-shaped bracket being fixed in the bar lug; and the bar being fixed on the support via the fork external shank arranged in the support hole and via a fastening member.

It is also provided for that the adjacent supports of the compression-distraction apparatus may be connected via the threaded rods and the repositioning assemblies, as used in combination, which designs are described above.

In order to achieve the technical effect in the repositioning assembly of the compression-distraction apparatus comprising sleeves which are hingedly connected via threaded shanks to the brackets fixed on the apparatus threaded rods via fastening members, the above sleeves are arranged on the threaded strut with the possibility of moving linearly and pivoting, the threaded strut is arranged with the possibility of moving linearly and pivoting in the slider hole and fixed via fastening members, the slider is provided on the side opposite to the said hole with a T-sectioned longitudinal slot, the end of the T-sectioned slot of the slider is provided with a plug with a recess, and a drive screw is arranged in the T-sectioned slot of the slider, which drive screw is fixed via the plug with recess, *according to this invention,* the slider is provided with a guide having longitudinal slots on its lateral sides, a longitudinal threaded channel and an external shank for fixation in the support hole via a fastening member, the guide is arranged on the ridges of the slider T-sectioned slot via the longitudinal slots, and the drive screw is also arranged in the longitudinal threaded channel of the guide.

Preferably, the slider of the repositioning assembly is made L-shaped.

Further, in order to achieve the technical effect the repositioning assembly of the compression-distraction apparatus, which comprises a threaded strut on which the first bracket is arranged for connection to an apparatus threaded rod via a fastening member, *according to this invention* is provided with a bar having two longitudinal protrusions on its opposite lateral sides, a lug on one its end, and a toothed cut piece on the other end, a fork provided with longitudinal recesses inside the end portions of both flanges and through holes made in both flanges for a wrench having a toothed ring, the bar being arranged inside the fork with the possibility of moving in such a way that its longitudinal protrusions cooperate with the recesses in the fork flanges, and the toothed cut piece cooperates with the toothed ring of the wrench arranged in the through holes of the fork flanges; the first bracket is made L-shaped with holes in its two flanges and is arranged on the threaded strut with the possibility of moving linearly and pivoting and is fixed via fastening members, the second L-shaped bracket is also arranged on the threaded strut and provided with a hole for the threaded strut on one flange and with an external threaded shank on the other flange for fixation of the bar in the support hole via a fastening member.

The invention is illustrated by the accompanying drawings, wherein:
Fig. 1 schematically shows a general isometric view of one embodiment of the compression-distraction apparatus;
Fig. 2 (a, b) schematically shows one embodiment of the repositioning assembly: a - disassembled, b - assembled;
Fig. 3 (a, b) schematically shows one embodiment of the repositioning assembly: a - disassembled (a turned view), b - assembled;
Fig. 4 (a, b) schematically shows one embodiment of the compression-distraction apparatus support in the form of a closed ring: a - disassembled, b - assembled;
Fig. 5 (a, b) schematically shows one embodiment of the compression-distraction apparatus support in the form of a open ring: a - disassembled, b - assembled.

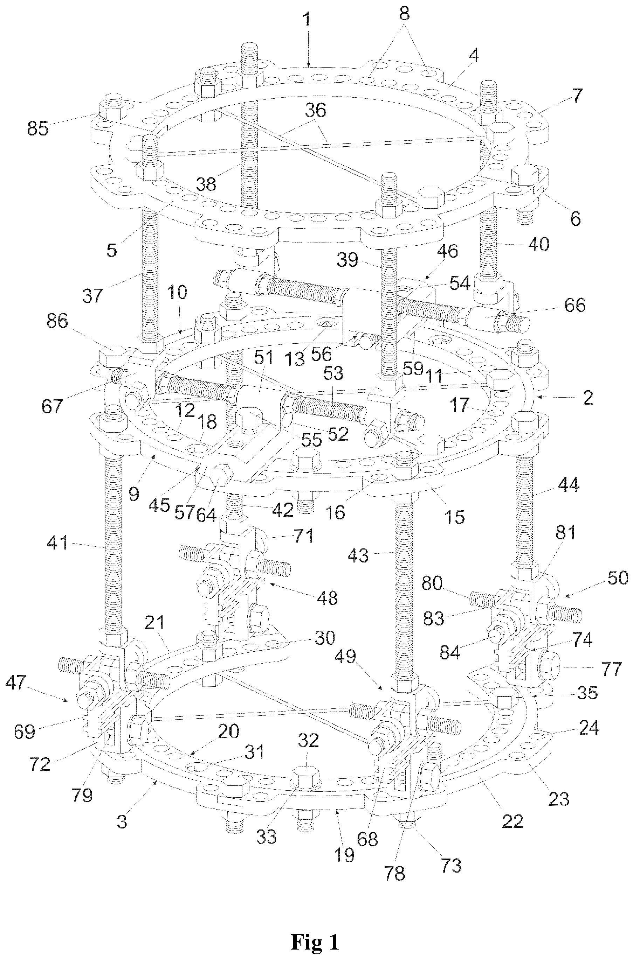

The compression-distraction apparatus (Fig. 1) comprises the supports 1, 2 and 3.

The support 1 is made in the form of a closed ring. The closed ring is made separable and consists of the arcuate segments 4 and 5 rigidly interconnected. The support 1 is provided with the lugs 7 on its external end face and the holes 8 on the arcuate segments 4 and 5 and the lugs 7. The matched stepped protrusions 6 are made on the ends of the arcuate segments 4 and 5. The stepped protrusions 6 of the arcuate segment 4 overlap the matched stepped protrusions 6 of the adjacent arcuate segment 5 via fastening members, in this case bolts, arranged in the holes 8, and nuts. These matched stepped protrusions 6 form the lugs 7 after connection.

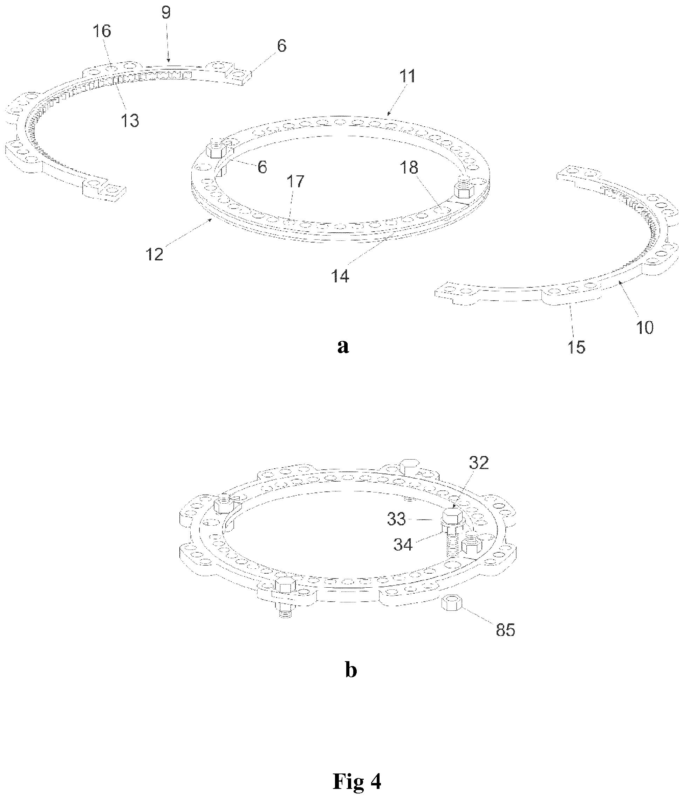

The support 2 is made in the form of a closed ring (Figs. 1, 4a, 4b). It comprises the outer part 9 and the inner part 11 that are coaxial. The coaxial outer part 9 of the support is made separable and consists of the arcuate segments 10 rigidly interconnected. The arcuate segments 10 of the support coaxial outer part are provided on their end faces with the lugs 15 having the holes 16. The ends of the arcuate segments 10 of the support coaxial outer part are provided with the stepped protrusions 6. They overlap the matched stepped protrusions 6 of the adjacent arcuate segment of the support coaxial outer part and are rigidly fixed via fastening members - bolts and nuts installed in the holes, thus forming the lugs 15. The arcuate segments 10 of the support coaxial outer part are provided with toothed rings 13 on their internal end faces. The coaxial inner part 11 is also made separable and comprises the arcuate segments 12. The coaxial inner part 11 is provided on its external end face with the slot 14 that corresponds to the toothed rings 13 of the arcuate segments 10 of the support outer part and that receives them during assembling. The segments 12 of the support coaxial inner part are provided with holes 17 and 18. The holes 18 are in communication with the slot 14. The ends of the arcuate segments 12 of the support coaxial inner part are provided with the stepped protrusions 6. They overlap the matched stepped protrusions of the adjacent arcuate segment of the support coaxial inner part and are rigidly connected via fastening members - bolts and nuts installed in the holes.

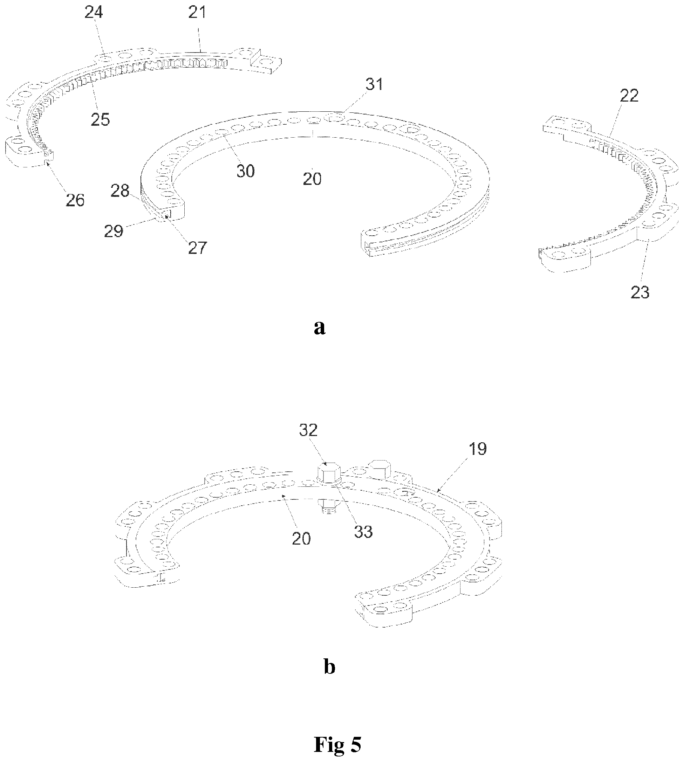

The support 3 (Fig. 1, 5a, b) is made in the form of an open ring. It comprises the outer part 19 and the inner part 20 that are arranged coaxially. The coaxial outer part 19 is made separable and consists of several arcuate segments, namely: two segments 21 and 22. The coaxial inner part 20 of the support 3 is made inseparable. The arcuate segments 21 and 22 of the support coaxial outer part 3 are provided on their external end faces with the lugs 23 having the holes 24 and on their internal end faces with the toothed rings 25. The slots 26 are made in the bases of the toothed rings 25 on both sides for forming T-shaped protrusions on the internal end face. The support coaxial inner part 20 is made inseparable. On its external end face it is provided with the T-sectioned slot 27 with the protrusions 28 and 29 facing each other and corresponding to the slots 26 in the bases of the toothed rings 25 of the arcuate segments of the support coaxial outer part 19. The support coaxial inner part 20 is provided with the holes 30 and 31. The hole 31 is in communication with the slot 27. The arcuate segments 21 and 22 of the support coaxial outer part are provided with the stepped protrusions 6 on their ends. The stepped protrusion 6 of the arcuate segment 21 overlaps the matched stepped protrusion 6 of the adjacent arcuate segment 22 of the support coaxial inner part and is rigidly connected via fastening members - bolts and nuts installed in the holes 24.

The wrenches 32 are arranged in the holes 18 and 31 of the coaxial inner parts of the supports 2 and 3, which are in communication with the slots 14 and 27 of the arcuate segments of the support coaxial inner parts (Figs. 4b and 5b). Each wrench 32 is made in the form of a bolt with the supporting collar 33 in the head base and with the toothed ring 34 along the periphery of the rod. The wrenches 32 are installed in the holes 18 and 31 with the possibility of cooperating with the toothed rings 13 and 25 of the segments of the coaxial outer parts of the supports 2 and 3 respectively.

The compression-distraction apparatus is provided with bone fixators that may be pins 36 and/or screwing rods (not shown in the drawings). The pins 36 are fixed on the supports via pin holders 35 arranged in the holes of the supports 1, 2 and 3.

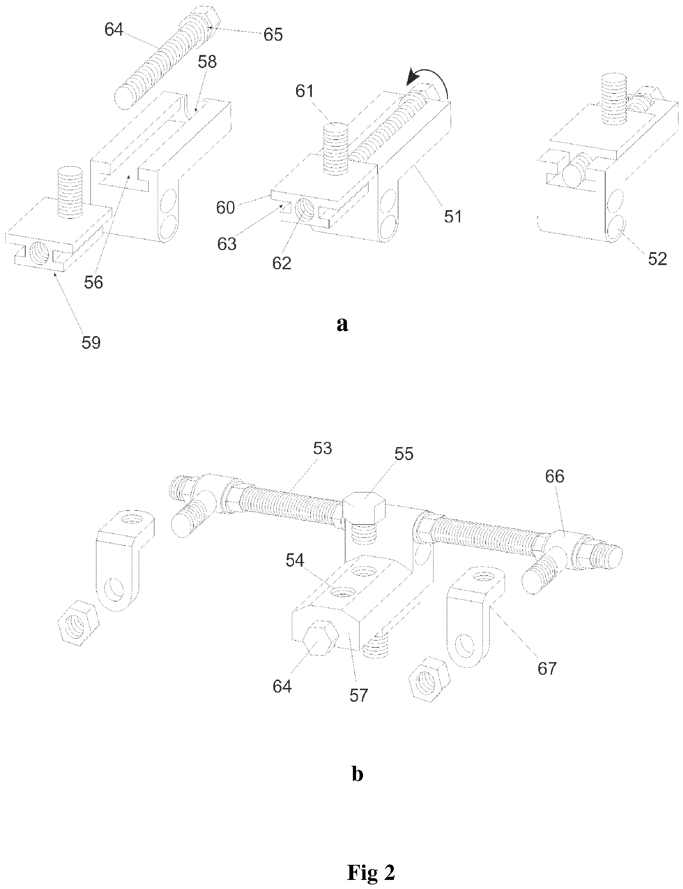

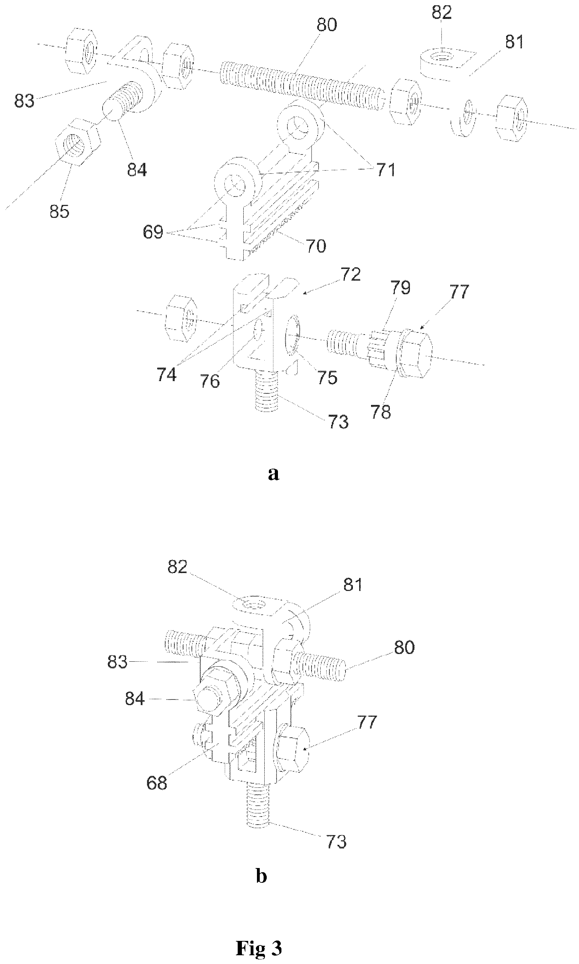

The supports 1 and 2 are interconnected via the threaded rods 37-40 and the repositioning assemblies 45 and 46 (Figs. 1, 2a, b), and the supports 2 and 3 are interconnected via the threaded rods 41-44 and the repositioning assemblies 47-50 (Figs. 1, 3a, b).

Each of the repositioning assemblies 45 and 46 comprises the L-shaped slider 51 with the holes 52 for the threaded strut 53 and the threaded holes 54 for the lock bolts 55. The threaded strut 53 is arranged with the possibility of moving linearly and pivoting in the hole 52 of the slider 51 and is fixed via nuts on both sides. The slider 51 has, on the side opposite to the hole 52, the T-sectioned longitudinal slot 56 with ridges, which is limited on its external end face by the plug 57 with the recess 58. The repositioning assembly is provided with the guide 59 comprising: the housing 60 with the threaded shank 61; the longitudinal threaded channel 62; and the longitudinal slots 63 made on its lateral sides and corresponding to the protrusions of the longitudinal T-sectioned slot 56 in the base of the slider 51. The guide 59 is arranged on the ridges of the longitudinal slot 56 via the longitudinal slots 63 on its lateral sides. The drive screw 64 is arranged in the T-sectioned slot 56 of the slider 51 and in the longitudinal threaded channel 62 of the guide; the slot 65 being made in the head basis of the drive screw for contacting the recess 58 of the slider plug 57 and fixed therein. The slider 51 is fixed on the support 2 via the guide outer shank 61 arranged in the hole 16 or 17 of the support and via a fastening member - nut.

The sleeves 66 provided with threaded shanks are arranged with the possibility of moving linearly and pivoting on the ends of the threaded strut 53 and fixed with nuts on both sides. The sleeves 66 are hingedly connected via threaded shanks and nuts to the L-shaped brackets 67 having holes on both flanges. The L-shaped brackets 67 are connected to the threaded rods 37 and 39 via nuts. The other ends of the threaded rods are fixed in the holes of the adjacent support 1 via nuts. The second repositioning assembly 5 is arranged in parallel to the first one. The L-shaped brackets 67 are connected to the threaded rods 38 and 40 via nuts. The other ends of the threaded rods are fixed in the holes of the adjacent support 1 via nuts.

The compression-distraction apparatus is also provided with the repositioning assemblies 47-50 (Figs. 1, 3a, b). each of them comprises the bar 68 having two longitudinal parallel protrusions 69 on its opposite lateral sides, the lugs 71 on one end face and the toothed cut piece 70 on the other end face. The bar 68 is fixed on the support 3 via the fork 72 with the external threaded shank 73 introduced into the hole 24 or 30 of the support outer or inner part respectively and fixed via a nut. The fork 72 inside the end portion of both flanges has the longitudinal parallel recesses 74 corresponding to the longitudinal parallel protrusions 69 of the bar 68 and the coaxially arranged through holes 75 and 76 in both flanges for the wrench 77. The wrench 77 is made in the form of a bolt having the supporting collar 78 in the head base and the toothed ring 79 along the rod periphery. The bar 68 is arranged inside the fork 72 with the possibility of moving so that its longitudinal protrusions 69 cooperate with the recesses 74 in the flanges of the fork 72, and the toothed cut piece 70 cooperates with the toothed ring 79 of the wrench 77 installed into the through holes 75 and 76 of the fork flanges. The first L-shaped bracket 81 and the second L-shaped bracket 83 are arranged ion the threaded strut 80 with the possibility of mobbing linearly and pivoting and are fixed via nuts.

The bracket 83 is provided with a hole for a threaded strut and with an external threaded shank 84 on the other flange. The external shank 84 is arranged in the lug 71 of the bar 69 and fixed via a nut. The threaded strut 80 is connected via the L-shaped bracket 81 having the threaded holes 82 on both flanges to one end of the threaded rod, and the other end is fixed in the hole 16 or 17 of the outer or inner part of the support 2, respectively. Similarly, the supports 2 and 3 are interconnected via the threaded rods 42-44 and the respective repositioning assemblies 48-50.

The individual parts are interconnected and fixed with the fastening nuts 85 and bolts 86.

The compression-distraction apparatus can be used as follows. Osteosynthesis of a damaged bone segment is carried out by passing the pins 36 through its distal, medial and proximal thirds in the conditions of an operating room after anesthetizing and treating the operative site, while proceeding from the clinical situation. After that, the apparatus is mounted, during which process the pins are fixed via the pin fixators 35 in the tensioned state on the supports 1, 2, 3 arranged at the respective levels. The support type for every level is selected proceeding from the clinical situation with due regard to localization of the damage or a planned level of violating the bone integrity during orthopedic reconstruction of the limb. Taking all this into account, the supports 2 and 3, which are assembled from the coaxial outer parts 9 and 19 and the coaxial inner parts 11 and 20, which are, in their turn, assembled from the arcuate segments 10 and 12 and 21 and 22, respectively, are arranged at the level adjacent to the sites where the bone integrity is violated, overlapping the stepped protrusions 6 and fixing via a nut. Supports in the form of open rings (support 3) are arranged near joints.

The supports 1, 2 and 3 are interconnected via the threaded rods 37-44 and the repositioning assemblies 45-50, the latter being arranged on the supports adjacent to the site of damage or purposeful violation of the bone integrity. If there is only one such site, then the supports that flank an intact segment are connected only via threaded rods.

When connecting the supports 1, 2, 3, the threaded rods 37-44 are fixed with the fastening nuts 85 in the holes 8 of the arcuate segments 4, 5 and the lugs 7 of the support 1, and the threaded shanks of the guides 59 of the repositioning assemblies 45 and 46 are fixed in the holes 16 of the lugs 15 or in the holes 17, respectively, of the arcuate segments 9 or 11 of the support 2 so that they are located in the vertices of the rectangles which centers coincide with the support centers, and their sides are parallel to each other and oriented relative to the frontal and sagittal planes.

The free ends of the threaded rods 37-40 are fixed in pairs via the sleeves 66 and the L-shaped brackets 67 on the ends of the threaded strut 53 of the repositioning assemblies 45 and 46, and the ends of the rods 41-44 are fixed in the threaded holes 82 of the brackets 81 of the threaded strut 80 of the repositioning assemblies 47-50. The repositioning assemblies 45 and 46 are used mainly in cases of osteosynthesis of oblique and spiral fractures; the repositioning assemblies 47-50 are recommended for use for other kinds of fractures as well as for carrying out orthopedic reconstruction of limbs. At each manipulation level both similar repositioning assemblies and a combination of the above repositioning assemblies may be used.

In order to move bone fragments by rotating the fastening nuts 85 fixing the ends of the threaded rods 37-44 on the supports 1 and 2, they are displaced along the required direction. The similar movement may be achieved by rotating the fastening nuts 85 fixing the ends of the threaded rods 41-44.

Angular deformities (displacements) of bone fragments on the frontal plane are eliminated by distraction along the rods on its concave side, e.g., along the rods 37 and 38 at one level and 41-42 at another level. While doing so, the rods on the convex side of a deformity, in this case the rods 39-40 at one level and the rods 43-44 at another level, are left in the neutral position or are used for carrying out compression, if inter-fragment diastasis is present on this side of the deformity. To do this, tightening of the nuts 85 fixing the positions of the L-shaped brackets 67 on the sleeves 66 of the repositioning assemblies 45 and 46 as well as the positions of the first L-shaped brackets 81 in the hole of the lug 71 of the bar 68 of the repositioning assemblies 47-50 should be loosened.

Angular deformities (displacements) of bone fragments in a sagittal plane are also eliminated by their distraction along rods on its concave side, for example, along the rods 37 and 39 at one level and 41 and 43 at another level. Rods located on the convex side of a deformity, in this case they are the rods 38 and 40 at one level and 42 and 44 at another level, are left in the neutral position, or compression is carried out along them, if inter-fragment diastasis is present on this side of the deformity. To do this, tightening of the nuts 85 fixing the positions of the threaded strut 53 in the holes of the slider of the repositioning assemblies 45 and 46 as well as the positions of the L-shaped brackets 81 at the threaded strut 80 of the repositioning assemblies 47-50 should be loosened.

Bone fragments may be moved in lateral directions with the use of the repositioning assemblies 45 and 46 by rotating the nuts 85 fixing the threaded strut 53 in the holes 52 of the sliders 51. Bone fragments may be moved in the front-rear directions by moving the slider 51 relative to the guide 59 by rotating the drive screw 64 in one or the other direction when the lock bolts 55 are loosened.

When eliminating lateral displacement with the use of the repositioning assemblies 47-50, the latter are oriented initially (during mounting of the apparatus) in a frontal plane in accordance with displacement to be made, then the fastening nuts 85 fixing the first L-shaped brackets 81 on the threaded struts 80 are rotated for moving them in the required direction, and then the nuts 85 are tightened again. If displacement in the opposite direction is necessary, the repositioning assemblies 47-50 should be rotated by 180°. To do this, the nuts 85 fixing the threaded rods 41-44 in the holes of the brackets 81 as well as the fork 74 on the support 3 are loosened, the repositioning assemblies 47-50 are rotated by 180°, and then their nuts are tightened again. In order to displace bone fragments in front-rear directions with the use of the repositioning assemblies 47-50, the nuts 85 fixing the wrench 77 in the holes of the fork 74 are loosened, and displacement in one or another direction is carried out by rotating the wrench head, and then the wrench 77 is fixed again by tightening the nut 85.

In order to perform rotational displacements in one or another side, the head of the bolt 32 arranged in the holes 18 and 31 of the arcuate segments of the support coaxial inner parts 2 and 3 is rotated, while turning the support coaxial inner and outer parts relative to each other.

After completing repositioning of bone fragments, restoring the biomechanical axis and length of the segment the apparatus is transferred to the stable fixation mode which is maintained until bone consolidation. The apparatus may be dismounted by reversing the above procedure.

The use of the proposed apparatus shows that it can ensure increased efficiency of its use due to the simplified constructions of the support connecting members and the possibility of selecting most optimal arrangement of the repositioning assemblies. The latter are oriented strictly along the axes of the Cartesian coordinate system, which crossing point being located in the fracture (osteotomy) epicenter, and two of them being orthogonal to the segment longitudinal axis, and the third one coincides with it. Owing to this, the apparatus can perform 6 independent movements - one linear and one rotational relative to each of them. This not only simplifies the bone fragments repositioning process to a maximum, but also excludes the necessity of re-mounting the apparatus. In a number of cases, e.g., in cases of lengthy fractures (osteotomies), the axis crossing point in the Cartesian coordinate system may be located at a level of proximal or distal fracture (osteotomy) center.

### FURTHER EMBODIMENTS OF THE INVENTION ARE:

1. A repositioning assembly for a compression-distraction apparatus, comprising sleeves that are hingedly connected via threaded shanks to brackets fixed on threaded rods of said apparatus via fastening members, wherein the sleeves are arranged on a threaded strut with the possibility of moving linearly and pivoting, the threaded strut is arranged with the possibility of moving linearly and pivoting in a slider hole and fixed via fastening members, the slider is provided with a T-sectioned longitudinal slot on the side opposite to the hole, the end of the slider T-sectioned slot being provided with a plug having a recess, a drive screw being arranged in the slider T-sectioned slot and fixed by the plug with the recess, *characterized in that* the slider is provided with a guide having longitudinal slots on its lateral sides, a longitudinal threaded channel and an external shank for fixing in a hole in the support via a fastening member, the guide being arranged on the ridges of the slider T-sectioned slot via the longitudinal slots, and the drive screw is also arranged in the longitudinal threaded channel of the guide.
2. The repositioning assembly for a compression-distraction apparatus according to embodiment 1, *wherein* the slider is made L-shaped.

## Claims

1. A compression-distraction apparatus, comprising: supports in the form of closed and/or open rings having holes and connected via threaded rods, bone fixators, and fastening members, at least one of said supports being composed of an inner part and an outer part, which parts being arranged with the possibility of being rotated relative to each other with a wrench having a toothed ring, the coaxial outer part of the support on its external end face being provided with lugs having holes and being formed of arcuate segments interconnected rigidly, ***characterized in that*** the arcuate segments of the support coaxial outer part are provided with toothed rings on its internal end face, and the support coaxial inner part is provided with a slot on its external end face, in which the toothed rings of the arcuate segments of the support coaxial outer part are arranged, the support coaxial inner part is provided with holes being in communication with the slot made in the support coaxial inner part and in which a wrench with a toothed ring is arranged with the possibility of cooperating with the toothed ring of the arcuate segment of the support coaxial outer part.

2. The compression-distraction apparatus according to Claim 1, ***characterized in that*** the supports are connected via threaded rods and repositioning assemblies.

3. The compression-distraction apparatus according to Claim 1 or 2, ***characterized in that*** the coaxial inner part of the said support is composed of the arcuate segments which ends are provided with stepped protrusions that overlap matched stepped protrusions of the adjacent arcuate segment of the support coaxial inner part and are rigidly interconnected via fastening members.

4. The compression-distraction apparatus according to Claim 1 or 2, ***characterized in that*** the support coaxial inner part in the form of an open ring is made inseparable, and the slot on its external end face is made T-sectioned with protrusions facing each other, the arcuate segments of the support coaxial outer part are provided with slots in the bases of the toothed rings on both sides for forming T-shaped protrusions corresponding to the said slot of the support coaxial inner part.

5. The compression-distraction apparatus according to any one of Claims 2-3, ***characterized in that*** the arcuate segments of the support coaxial outer part are provided on their ends with stepped protrusions having holes, which protrusions overlap matched stepped protrusions with holes of the adjacent arcuate segment of the support coaxial outer part for forming lugs and rigidly connected via fastening members.

6. The compression-distraction apparatus according to any one of Claims 2-5, ***characterized in that*** the repositioning assemblies are made in the form of sleeves that are hingedly connected via threaded shanks to L-shaped brackets fixed on the apparatus threaded rods via fastening members, said sleeves being arranged on a threaded strut with the possibility of moving linearly and pivoting, and the threaded strut being arranged with the possibility of moving linearly and pivoting in a hole made in a slider, a slider being provided on the side opposite to the hole with a T-sectioned longitudinal slot on which ridges a guide is arranged via longitudinal slots made on its lateral sides, the end of the slider T-shaped slot being provided with a plug having a recess, a drive screw being arranged in the slider T-shaped slot and in a longitudinal threaded channel made in a guide, said drive screw being fixed via the plug with the recess, and the slider being fixed on the a support via an external shank made on the guide and arranged in a hole of the support and via a fastening member.

7. The compression-distraction apparatus according to any one of Claims 2-5, ***characterized in that*** the repositioning assemblies each comprise: a threaded strut; a first and a second L-shaped brackets that are arranged on the threaded strut with the possibility of moving linearly and pivoting and are fixed via fastening members, the first L-shaped bracket being provided with threaded holes in both flanges and connected to a threaded rod of the apparatus via a fastening member; a bar having two longitudinal protrusions on its opposite lateral sides, a lug on one end face and a toothed cut piece on the other end face; a fork provided with longitudinal recesses inside the end portions of both flanges and coaxial through holes in both flanges for a wrench having a toothed ring, the bar being arranged within the fork with the possibility of moving so that its longitudinal protrusions cooperate with the recesses in the fork flanges, and the toothed cut piece cooperates with the toothed ring of the wrench arranged in the through holes of the fork flanges; the second L-shaped bracket being provided with a hole for a threaded strut on one flange and with an external threaded shank on the other flange, and the threaded shank of the second L-shaped bracket being fixed in the bar lug, the bar being fixed on a support via the fork external shank installed in the hole made in the support and via a fastening member.

8. The compression-distraction apparatus according to any one of Claims 2-5, ***characterized in that*** the adjacent supports are connected via the threaded rods and repositioning assemblies comprising sleeves that are hingedly connected via threaded shanks to brackets fixed on threaded rods of said apparatus via fastening members, wherein the sleeves are arranged on a threaded strut with the possibility of moving linearly and pivoting, the threaded strut is arranged with the possibility of moving linearly and pivoting in a slider hole and fixed via fastening members, the slider is provided with a T-sectioned longitudinal slot on the side opposite to the hole, the end of the slider T-sectioned slot being provided with a plug having a recess, a drive screw being arranged in the slider T-sectioned slot and fixed by the plug with the recess, ***characterized in that*** the slider is provided with a guide having longitudinal slots on its lateral sides, a longitudinal threaded channel and an external shank for fixing in a hole in the support via a fastening member, the guide being arranged on the ridges of the slider T-sectioned slot via the longitudinal slots, and the drive screw is also arranged in the longitudinal threaded channel of the guide.
